(19) **Europäisches Patentamt**
**European Patent Office**
**Office européen des brevets**

(11) **EP 4 559 437 A1**

(12) **EUROPEAN PATENT APPLICATION**

(43) Date of publication:
**28.05.2025   Bulletin 2025/22**

(21) Application number: **23211455.3**

(22) Date of filing: **22.11.2023**

(51) International Patent Classification (IPC):
**A61F 2/30** *(2006.01)*   **A61B 34/10** *(2016.01)*
**A61F 2/44** *(2006.01)*

(52) Cooperative Patent Classification (CPC):
**A61F 2/30942; A61B 34/10; A61F 2/4455;**
A61B 2034/105; A61B 2034/108; A61F 2002/3092;
A61F 2002/3093; A61F 2002/30948;
A61F 2002/30952; A61F 2002/30955;
A61F 2002/30985

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **ETH Zurich**
  **8092 Zurich (CH)**
• **Denmarks Tekniske Universitet**
  **2800 Kongens Lyngby (DK)**
• **Schulthess Klinik**
  **8008 Zurich (CH)**

(72) Inventors:
• **Smit, Thijs**
  **4058 Basel (CH)**
• **Helgason, Benedikt**
  **8046 Zurich (CH)**
• **Ferguson, Stephen J.**
  **8340 Hinwil (CH)**
• **Aage, Niels**
  **2200 Copenhagen (DK)**
• **Haschtmann, Daniel**
  **8700 Küsnacht (CH)**

(74) Representative: **Braunpat AG**
  **Peter Merian-Strasse 28**
  **4052 Basel (CH)**

(54) **METHOD FOR DESIGNING PATIENT-SPECIFIC INTERBODY IMPLANT MODELS AND METHOD FOR TRAINING AN ARTIFICIAL INTELLIGENCE MODEL FOR DESIGNING PATIENT-SPECIFIC INTERBODY IMPLANT MODELS**

(57)     The present invention relates to a computer-implemented method (100) for designing an optimized patient-specific implant model (1) in view of its manufacturing comprising the following steps:

a. define in a patient-specific representation of the region to receive the implant (3), at least a first bone domain (3a), a second bone domain (3b) and between the first and second bone domains an implant domain (3c), wherein the first bone domain (3a), the second bone domain (3b) and the implant domain (3c) form the optimization domain;

b. create an initial implant model (1) that at least partially fills the implant domain (3c), wherein the boundaries of the initial implant model (1) are designed to fit the boundaries of the first and second bone domains (3a,3b) facing the implant domain (3c);

c. optimize the implant model (1) by applying optimization, advantageously topology optimization or artificial intelligence-based optimization, until at least one of several termination criteria is reached, wherein the optimization is based on the mechanical response of at least of one of the first bone domain or the second bone domain (3a,3b) to mechanical load, in particular the subsidence risk.

Fig. 2

EP 4 559 437 A1

**Description**

Technical field of the invention

The present invention relates to the field of interbody implants. More specifically, the present invention concerns a method for designing patient-specific interbody implant models as well as a method for training an artificial intelligence model for designing patient-specific interbody implant models. The project leading to this application has received funding from the European Union's Horizon 2020 research and innovation programme under the Marie Sktodowska-Curie grant agreement No 812765.

State of the art

The goal of every orthopaedic implant is to stabilize the treated domain by fixating the treated bones relative to each other. This allows the bone healing process to start and bridge the fractured elements by natural bone growth. A variety of implants are commonly used by orthopaedic surgeons like spine implants, hip implants, knee implants and implants to bridge critically sizes bone defects in bones.

Lower back pain is for instance one of the most common physical complaints in the world. Degenerative Disc Disease (DDD) can cause lower back pain, a syndrome in which the intervertebral disc (IVD) is degraded by wear, tear and biological processes. A common procedure for treating advanced DDD, and other spinal disorders, is spinal fusion. The spinal fusion implant replaces the IVD and aims to restore the space between the vertebrae to the IVD's original height. This relieves pain by reducing pressure on the nerves. Furthermore, the implant system stabilizes and fixes the position of the two-vertebra adjacent to the IVD and therefore supports these vertebrae to fuse together. Implanting a spinal fusion implant will change the load path through the spine. This is because the geometry and material properties of the implant are different from those of the native IVD. For example, the stiffness of a titanium fusion implant is higher than the stiffness of the IVD. Furthermore, the contact area of the implant with the vertebra is smaller than the original contact area between the IVD and adjacent vertebra. As a result, the vertebrae are subject to changed mechanical stresses and stress peaks, and changes strains and strain peaks, following the intervention, which can lead to a phenomenon called subsidence.

Subsidence can lead to reduced space between the adjacent vertebrae which can increase pain due to increased pressure on the nerves. Clinical follow-up studies show that between 10% and 76% of fusion implants will subside, depending on the definitions used. A revision surgery is currently the only solution for addressing severe subsidence. Revision surgery rates are reported to be around 20%. This impacts the patient's well-being and is costly for the health care systems.

The methods to design fusion implants known in the prior art are either manual methods, i.e. based on the knowledge and experience of an orthopaedic physician and/or mechanical engineer who designates the implant, or methods of which the implants are designed automatically by a computer. The known computer implemented methods do however not achieve optimal results, and it is often necessary, as mentioned above, to operate on the patient again to change the implant.

Moreover, methods to design Anatomically Conforming Devices (ACDs) where the cage's geometry was adapted to match the patient's vertebral endplate shape, using Computed Tomography (CT) scans as input are known. These ACDs were suggested to reduce subsidence risk compared to Off-The-Shelf (OTS) cages. Current ACDs are designed to match the patient's anatomy but ignore the load bearing capacity of the adjacent bone structures. Previous studies employing optimizing OTS cages do not incorporate patient-specific information.

It is therefore evident that there exists a need for a method for designing patient-specific implants that reduce the risk of revision surgery.

Summary of the invention

Thus, the object of the present invention is to propose a novel method with which the above-described drawbacks are completely overcome or at least greatly diminished.

An object of the present invention is in particular to propose a computer-implemented method for designing an optimized patient-specific implant model.

An object of the present invention is also a method for training an artificial intelligence model for the design of optimized implant models.

According to the present invention, these objects are achieved in particular through the elements of the two independent claims. Further advantageous embodiments follow moreover from the dependent claims and the description.

In particular, the objects of the present invention are achieved by a computer-implemented method for designing an optimized patient-specific implant model in view of its manufacturing comprising the following steps:

a. define in a patient-specific representation of the region to receive the implant, at least a first bone domain, a second bone domain and between the first and second bone domains an implant domain, wherein the first bone domain, the second bone domain and the implant domain form the optimization domain;

b. create an initial implant model that at least partially fills the implant domain, wherein the boundaries of the initial implant model are designed to fit the boundaries of the first and second bone domains facing the implant domain;

c. optimize the implant model by applying optimization, advantageously topology optimization or artificial intelligence-based optimization, until at least one of several termination criteria is reached, wherein the optimization is based on the mechanical response of at least of one of the first bone domain or the second bone domain to mechanical load, in particular the subsidence risk.

This method allows to obtain implant models which, when manufactured and implanted in the patient's body, reduce the risk of having to re-operate the patients. By optimizing the implant model on the basis of the mechanical response of the bone domains to mechanical load, it is possible to integrate in the design process of the implant model the mechanical response of the bone domains to the mechanical loads that are expected in the life of the patient. Therefore, it is advantageous that the applied mechanical loads are patient specific, i.e. they are advantageously dependent for instance on the weight, height and age of the patient. In addition, the present method allows to produce implants that promote fusion between the implants and the bone between which they are placed.

According to a first preferred embodiment of the present invention, the implant model is in step c iteratively optimized by applying topology optimization until at least one of several termination criteria is reached, wherein in each iteration the mechanical response of at least of one of the first bone domain or the second bone domain to mechanical load, in particular the subsidence risk, is determined and used for optimizing the implant model. Topology optimization offers distinct advantages over other types of optimization methods in engineering design, though it should be noted that the choice of optimization method often depends on the specific requirements and constraints of a given project. One of the primary advantages is the material savings it can offer. By redistributing material in an optimal manner, it often produces designs that are lighter yet just as strong or even stronger than conventional designs. Topology optimization often results in better-performing structures in terms of various mechanical attributes like stiffness, strength, and heat dissipation. Furthermore, topology optimization is not constrained by traditional design rules and thus can explore a wider design space. Moreover, topology optimization can be integrated into various manufacturing methods, including additive manufacturing, to produce complex optimized structures that were previously not feasible to manufacture. Finally, topology optimization can easily be set up to account for uncertainties in material properties, loading conditions, or other variables, thereby creating more robust designs.

According to a further preferred embodiment of the present invention, the mechanical load comprises shear loads, rotational loads, tension and/or compression load. This allows for taking into account, in the design process, all the relevant loads that occur in the life of the patients.

According to a further preferred embodiment of the present invention, the mechanical load comprises daily living loads and loads of higher intensity than daily living loads, wherein the intensity of the daily living loads are determined based on the weight of the patient. This allows for taking into account more precisely the relevant loads that occur in the life of the patients.

In another preferred embodiment of the present invention, the value of the objective function comprises the compliance, stiffness, or load bearing capacity of the optimization domain. This allows to obtain optimized implant models that are applied in different situations in the body of the patient. When the stiffness is the most important factor for one type of implants, the load bearing capacity and the compliance are more important for other types of implants.

According to another preferred embodiment of the present invention, the strain response, in particular the minimum and maximum principal strains, or the stress response, in particular principal stresses or von Mises stress, or the energy response, in particular strain energy, of the first bone domain or/and of the second bone domain are constrained. This allows for constraining the maximum strain, stress or energy response that the bone domains can be subject to.

In a further preferred embodiment of the present invention, the minimum and maximum volume or local volume of the implant domain is constrained. This allows to create a porous implant model structure and to control the porosity and pore-size distribution to be favourable for bone in-growth.

In a further preferred embodiment of the present invention, the topology optimization formulation comprises a manufacturability constraint for the implant domain. This is advantageous since it promotes the manufacturability of the designed implants by a metal powder bed fusion process.

According to another preferred embodiment of the present invention, the minimum diameter of the implant domain structures, for example struts, is constrained. This improves manufacturability and the integrity of the implant structure.

According to yet another preferred embodiment of the present invention, the patient-specific image is used to identify at least one tissue characteristic at one or more locations inside the first bone domain and/or the second bone domain, wherein the at least one tissue characteristic comprises tissue density, tissue elasticity, tissue compliance, tissue stiffness and/or tissue strength, and wherein the at least one tissue characteristic is used to determine the mechanical response of the first bone domain and/or the second bone domain to mechanical load, and wherein the implant model is advantageously configured to contact a portion of the first bone domain and of the second bone domain having a relatively higher tissue density compared to non-contacted portions of the first and second bone domains. This allows to take into account the patients specific characteristic of the bone domains in the implant design process.

In all the embodiments of the present invention, the patient-specific image is advantageously a Computed Tomography (CT) image, for example, an X-ray image, in particular an X-ray CT image, a positron emission tomography (PET) image, a magnetic resonance imaging (MRI) image, a bone density scan image, such as a DEXA image, or any patient-specific image that provides bone information.

According to a further preferred embodiment of the present invention, the method comprises a step for generating manufacturing information for manufacturing the patient-specific implant. This allows for manufacturing the implant based on the optimized implant model for instance by additive manufacturing.

In a specific embodiment of the present invention, the patient-specific implant is a spinal implant, in particular a spinal fusion implant, and the first bone domain and the second bone domain represent two adjacent vertebrae and wherein the implant is advantageously a spinal fusion implant cage.

Advantageously, the method of the present invention comprises a step of assessing the risk of subsidence of the optimized implant model in at least one of the bone domains.

According to a second aspect of the present invention, the objects of the present invention are achieved by a method for training an artificial intelligence model for the design of optimized implant models, said method comprising the step of utilizing at least one optimized implant model, said optimized implant model being obtained in accordance with the method as defined in any one of claims 1 to 14, wherein said artificial model is a neural network, preferably a convolutional neural network, and wherein said optimized implant models are specifically configured for spinal implants, more preferably spinal fusion implants. Indeed, the method of the present invention directed towards optimized implant model design can be used to create data that can be used to train an artificial intelligence model which can then, when sufficiently trained, create ab initio optimized implant models that do not need further optimization.

In a further aspect the present invention relates to a data processing device comprising means for carrying out the method of claim 1.

In a further aspect the present invention relates to a computer program product comprising instructions which, when the program is executed by a computer, cause the computer to carry out the method of claim 1.

In another aspect the present invention relates to a computer-readable storage medium comprising instructions which, when executed by a computer, cause the computer to carry out the method of claim 1.

Short description of the figures

Figure 1 is a schematic side view of two vertebrae adjacent to a spinal fusion implant;

Figure 2 illustrates the workflow of the method of the present invention;

Figure 3a and b show the segmentation of a patient specific image and a surface representation of the segmented vertebrae;

Figure 4a-4d show CAD models of an OTS spinal fusion implant and of the ACD implant model;

Figure 5a-5b illustration of the optimization domain and of the mechanical loads applied to the model;

Figure 6a-6b illustration of the mechanical load applied for subsidence risk assessment;

Figure 7a-7h show different view of two optimized implant models; and

Figure 8 histograms for the subsidence risk assessment for both patients for the OTS, ACD and TOD.

Preferred embodiment

A preferred embodiment of the present invention is illustrated below with respect to the design of patient-specific spinal fusion implants. However, it is expressly noted that the invention's applicability extends beyond spinal fusion implants and encompass every implant, in particular every interbody implant.

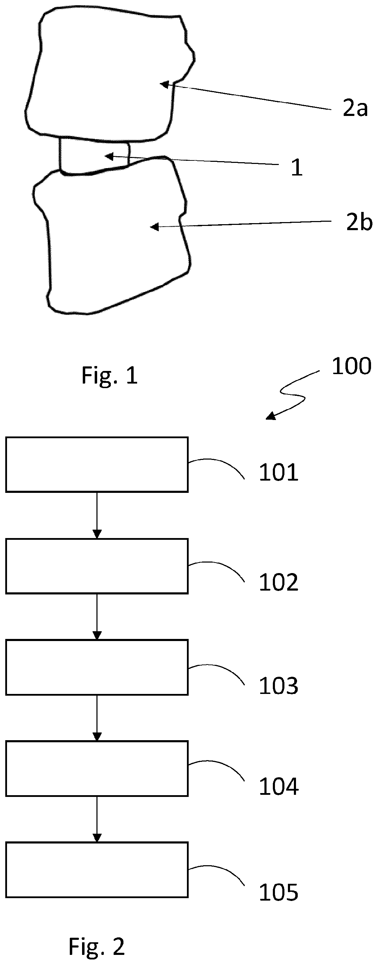

Figure 1 shows a schematic illustration of a spinal implant 1 placed between two vertebrae 2a, 2b. The preferred embodiment of the method of the present invention illustrated in details below has the goal of designing an optimized implant 1.

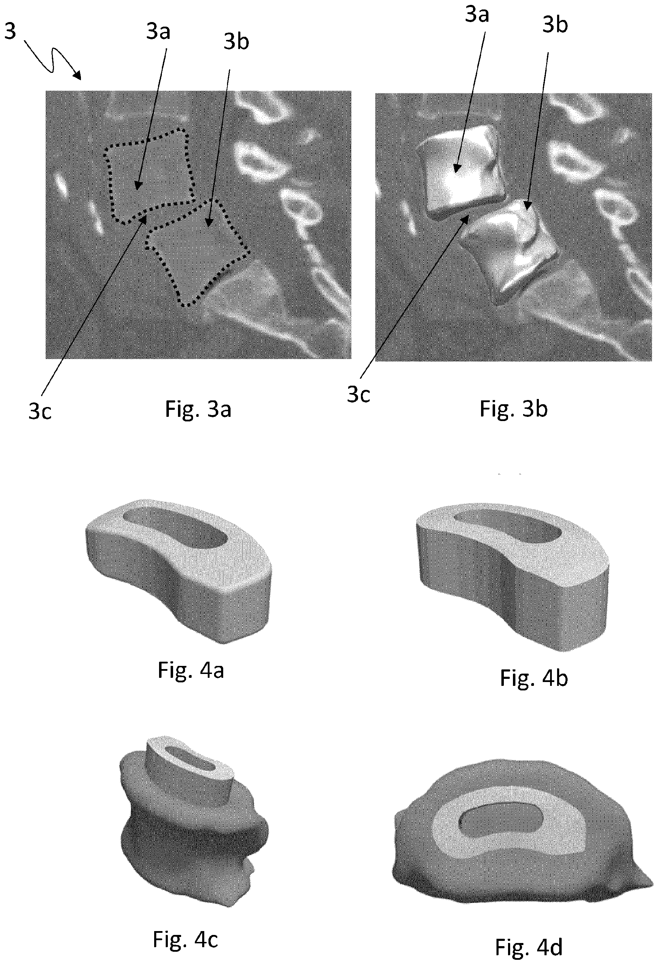

Figure 2 illustrates the workflow of a preferred embodiment 100 of the present invention. In step 101, at least a first bone domain 3a, a second bone domain 3b and between the first and second bone domains an implant domain 3c are defined in a patient specific image or representation 3 (see Figure 3) of the region to receive the implant. Such an image or representation for example can be a computed tomography (CT) image, in particular a X-ray CT image, a positron emission tomography (PET) image, a magnetic resonance imaging (MRI) image, a bone density scan image, such as a DEXA image, or any patient-specific image that provides bone information.

In step 102, an initial implant model that at least partially fills the implant domain is created, wherein the boundaries of the initial implant model are designed to fit the boundaries of the first and second bone domains facing the implant domain.

In step 103, the implant model is, in the preferred embodiment of the present invention, optimized by topology optimization until at least one of several termination criteria is reached, wherein in each iteration the mechanical response of at least of one of the first bone domain or the second bone domain to mechanical load, in particular the subsidence risk, is determined and used for optimizing the implant model.

In step 104, an assessment of the subsidence risk of the optimized implant model is performed and in step 105 an implant is manufactured, for instance by means of 3D-printing, on the basis of the optimized implant model.

Details of the method of Figure 1 and results obtained by applying this method on two patients are presented below.

Pre-operative CT scans with clinical resolution (voxel sizes of about: 0.30 mm × 0.30 mm × 0.50 mm) of two human Functional Spinal Units (FSU), from two female donors were acquired. These patients were diagnosed with Degenerative Spondylolisthesis and treated with a TLIF procedure. The patients had no history of spinal diseases, spinal trauma, or spinal deformity. Based on the available T-score (deviation score that compares an individual's bone density to the average score for a healthy 30-year-old adult of the same sex derived here from DXA scan) and the calculated mean integral volumetric bone mineral density (integral vBMD), patient 1 was classified as osteopenic and patient 2 was classified as having normal bone quality.

Segmentation of the image into a first bone domain, a second bone domain and an implant domain is performed manually using the open-source software MITK-GEM version 2017.05.0 (Figure 3a and Figure 3b). Figure 3a shows the results of the segmentation and Figure 3b shows a surface representation of the segmented vertebrae. Furthermore, a material mapping function is applied to obtain the Young's moduli $E(\rho_{app})$ of the bone domains.

A commonly used Off-The-Shelf (OTS) commercial cage for a Transforaminal Lumbar Interbody Fusion (TLIF) procedure with a height of 9 mm was reengineered into a CAD model (Figure 4a). This CAD model is used as the basis for the Anatomically Conforming Devices (ACD) and the Topologically Optimized Devices (TOD) design domain by extruding the top and bottom surfaces to match the endplates of the patient's vertebrae (Figure 4b).

Intervertebral disc material and cartilaginous endplates are assumed to be at least partially removed during the surgical procedure. The implants, here spinal fusion implants, were placed anteriorly and across the mid-line in an optimal position and restoring spinal alignment as much as possible (Figure 4c and Figure 4d). The implant placement is checked by a spine surgeon according to standard surgical fusion techniques for the TLIF approach. The ACD model is used as a design domain for the Topology Optimization (TO) process. This is ensuring that the contact areas between the cages and endplates are equal for all models to allow for a fair comparison.

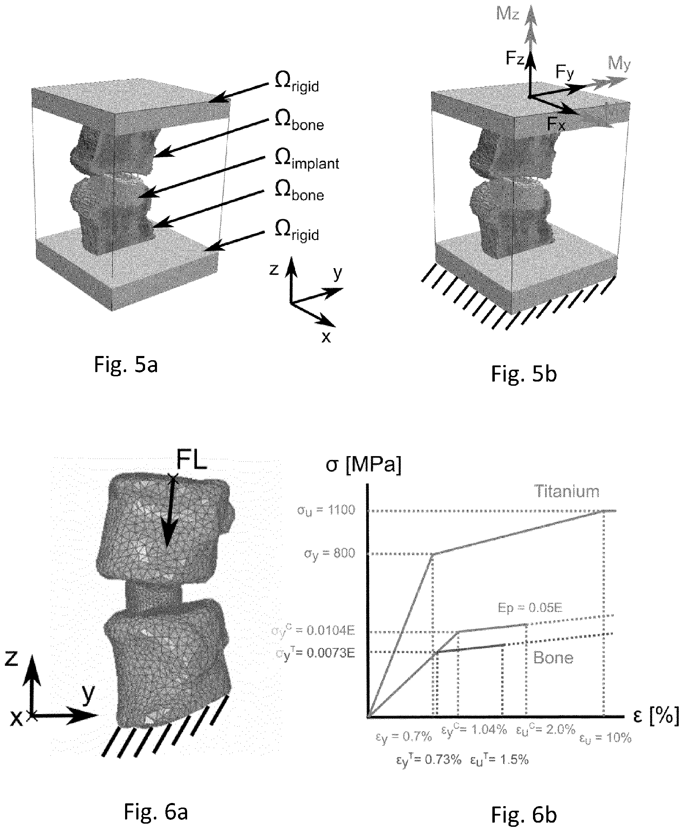

Having processed the patient-specific data, the following sections present how TO was applied to achieve optimized implant design models. The optimization domain $\Omega$ is composed of an implant domain ($\Omega_{implant}$), a bone domain ($\Omega_{bone}$, comprising both bone domains above) and advantageously a rigid domain ($\Omega_{rigid}$) to apply mechanical stress as it will be explained below (Figure 5a). $\Omega_{bone}$ contains the bone structures and patient-specific material mapping which are obtained from the patient's CT scans. $\Omega_{implant}$ is the design domain that is used in the TO process.

A full-scale topology optimization formulation was implemented using a large-scale TO framework which can design structures with high-resolution. The formulation follows the density approach with a linear elastic Finite Element (FE) model, assuming small strains. The Solid Isotropic Material and Penalization method (SIMP) was used and as optimizer, the Method of Moving Asymptotes (MMA) was integrated. Domain $\Omega$ was discretized using a structured grid of 1.728 million hexahedron elements with a mesh size of 0.45 mm. A resampling was performed to adjust the CT scan resolution to $\Omega$ using SimpleITK version 2.1.1.2. The bone-implant interface is assumed to be fully bonded. A Partial Differential Equation (PDE) filter is used to avoid numerical artifacts.

Titanium was selected as implant material as it is widely used in orthopaedic implants. Furthermore, the known metal powder bed fusion processes have sufficiently high-resolution to additively manufacture the optimized implants.

A two-stage design process was employed. In the first stage the implant was optimized for daily-living loading conditions. These daily-living loads, because they are relatively small, cause strains in the adjacent bone structures and the implant in the linear regime. For the proposed optimization problem to be using a linear elastic finite element (FE) model is a reasonable and sufficient assumption as the adjacent bone structures and implant do not undergo plastic deformation under daily-living loading in the optimization process. In the second stage, a verification was performed in a commercial finite element analysis software, where the implants were loaded with extreme loading, to evaluate and compare the implant's subsidence risks. In this stage a non-linear FE model and material model was used to quantify the post-yield effects and subsidence risk.

Six load-cases (axial compression, lateral shear, posterior-anterior shear, flexion, lateral bending, and axial rotation) were used for the daily-living loads (Figure 5b). For each load-case a recorded daily-living loading, from a matching patient based on body weight was used. Loads were applied on nodes at the top of $\Omega$ while nodes on the bottom of $\Omega$ were fixed in all directions (Figure 5b).

The most important function of the spinal fusion cage is to provide primary stability to the treated segment allowing the two vertebrae to fuse together. From an optimization perspective this means that the stiffness shall be maximized (which correspond to minimizing compliance) of the bone-implant system for all load-cases (optimization objective) but avoid over-loading of the adjacent bone structures. To this end, the maximum and minimum principal strains of the bone structures in the adjacent vertebrae ($\Omega_{bone}$), are constrained (optimization constraints). Apart from strain constraints, a local volume constraint on $\Omega_{implant}$ was advantageously used to create a porous implant structure and to control the porosity and pore-size distribution to be favourable for bone in-growth. To promote manufacturability of the designed cages by a metal powder bed fusion process, a minimum strut diameter control was used via a robust formulation (see below). Additionally, a connectivity constraint was added to the formulation and applied to $\Omega_{implant}$, to create interconnected pores which facilitates bone in-growth and the removal of unsolidified powder in the additive manufacturing process.

To ensure that the optimized designs were robust with regards to manufacturing as well as strain levels in $\Omega_{bone}$, a so-called robust topology optimization formulation was employed. This amounts to the application of a modified robust optimization formulation, based on the three-field density projection; the eroded $x^e$, the intermediate $x^i$ and the dilated design $x^d$. The compliance minimization problem with maximum and minimum principal strain constraints, local volume constraint and connectivity constraint in discrete form is written as:

$$\min_{x} \sum_{k=1}^{lc} f(x^e)_k \quad in \quad \Omega$$

1, objective function

$$subject\ to$$

$$\sum_{k=1}^{lc} g(x^d)_k \leq 0.0 \quad in \quad \Omega_{bone}$$

2, maximum principal strain constraint

$$\sum_{k=1}^{lc} q(x^d)_k \leq 0.0 \quad in \quad \Omega_{bone}$$

3, minimum principal strain constraint

$$v(x^d) \leq 0.0 \quad in \quad \Omega_{implant}$$

4, local volume constraint

$$c(x^d) \leq 0.0 \quad in \quad \Omega_{implant}$$

5, connectivity constraint

where $lc$ represents the six load-cases with $k = 1 \ldots 6$, $f$ the objective function, $g$ and $q$ the constraints on the maximum and minimum principal strain response in the $\Omega_{bone}$, respectively, $v$ the local volume constraint and c the connectivity constraint on $\Omega_{implant}$.

The objective is to maximize the stiffness (minimize compliance) of the bone-implant system in $\Omega$ for all load-cases. The objective function is evaluated using the eroded design $x^e$ because this realization is producing the worst-case objective values. The objective function is defined as the strain energy corresponding to load-case $k$:

$$f(x^e)_k = (u^T K u)_k$$

where $u$ is the displacement field corresponding to load-case $k$, and $K$ the stiffness matrix. The maximum and minimum principal strains are calculated using:

$$pe\_max = \frac{2}{\sqrt{3}}\sqrt{I_2}\sin\left(\theta + \frac{2\pi}{3}\right) + \frac{I_1}{3}$$

$$pe\_min = \frac{2}{\sqrt{3}}\sqrt{I_2}\sin\left(\theta - \frac{2\pi}{3}\right) + \frac{I_1}{3}$$

where

$$\theta = \frac{1}{3}\sin^{-1}\left(-\frac{3\sqrt{3}}{2}\frac{I_3}{I_2^{3/2}}\right), -\frac{\pi}{6} \leq 0 \leq \frac{\pi}{6}$$

The strain invariants $I_1$, $I_2$ and $I_3$ are calculated using:

$$I_1 = \mathbf{M}\,\boldsymbol{\varepsilon}$$

$$I_2 = \frac{1}{3}\boldsymbol{\varepsilon}^T\mathbf{V}\boldsymbol{\varepsilon}$$

$$I_3 = s_{11}\,s_{22}\,s_{33} + 2s_{23}s_{13}\,s_{12} - (s_{11}s_{23}^2 + s_{22}s_{13}^2 + s_{33}s_{12}^2)$$

where $s_{ij}$ $i,j = 1,..,3$ are the components of the deviatoric strain tensor and $\varepsilon$ the element strain tensor $[\varepsilon_{11}\,\varepsilon_{22}\,\varepsilon_{33}\,\varepsilon_{23}\,\varepsilon_{13}\,\varepsilon_{12}]^T$, which is obtained by averaging the contributions from the 8 integration points per element.
The deviatoric strain tensor $\mathbf{s}$ is computed by:

$$\mathbf{s} = \boldsymbol{\varepsilon} - \boldsymbol{\varepsilon}_{hyd}$$

$$\boldsymbol{\varepsilon}_{hyd} = \frac{\varepsilon_{11} + \varepsilon_{22} + \varepsilon_{33}}{3}$$

Furthermore, $\mathbf{M} = [111000]$ and

$$\mathbf{V} = \begin{bmatrix} 1 & -1/2 & -1/2 & 0 & 0 & 0 \\ -1/2 & 1 & -1/2 & 0 & 0 & 0 \\ -1/2 & -1/2 & 1 & 0 & 0 & 0 \\ 0 & 0 & 0 & 3 & 0 & 0 \\ 0 & 0 & 0 & 0 & 3 & 0 \\ 0 & 0 & 0 & 0 & 0 & 3 \end{bmatrix}$$

The constraint values, before aggregation are introduced using:

$$\Lambda_{max} = \frac{pe\_max}{pe\_max_{lim}} - 1.0$$

and

$$\Lambda_{min} = \frac{pe\_min}{pe\_min_{lim}} - 1.0$$

Here, $\Lambda_{max}$ and $\Lambda_{min}$ are aggregated using a p-mean aggregation function to obtain g and q:

$$g = \frac{1}{n}\left(\sum_{1}^{n}\Lambda_{max}{}^{p}\right)^{1/p}$$

$$q = \frac{1}{n}\left(\sum_{1}^{n}\Lambda_{min}{}^{p}\right)^{1/p}$$

The constraints, g and q, are calculated using the dilated design $x^d$ as this realisation is producing the worst-case strain states in $\Omega_{bone}$. A maximum principal strain limit $pe\_max_{lim}$ of 0.73% and a minimum principal strain limit $pe\_min_{lin}$ of -1.04% were used as limits for the strain states in $\Omega_{bone}$.

To ensure mesh-independent solutions to the posed optimization problem, a length scale was advantageously introduced. This is obtained by filtering schemes. A PDE filter is converting the mathematical design variables $x$ to the filtered design variables $\tilde{x}$. A projection was used to ensure a solid/void design. The projection resulted in the physical design variable $\hat{x}$ with,

$$\widehat{x} = \frac{\tan^{-1}(\beta\mu) + \tan^{-1}(\beta(\tilde{x} - \mu))}{\tan^{-1}(\beta\mu) + \tan^{-1}(\beta(1 - \mu))}$$

with $\beta$ the projection parameter and $\mu$ the threshold parameter corresponding to realization $\mu^d$, $\mu^i$ and $\mu^e$. $\beta = 1$ was used at the start of the optimization and increased to a maximum of $\beta = 64$.

The use of the robust formulation results in a controllable minimum strut diameter which improves manufacturability and the integrity of the implant structure. Having at least 4 elements through the cross section of a single strut was targeted (minimum length-scale $r_{min}$). Therefore, a filter radius $r_{fil}$ was set to 0.40. $\mu^e = 0.65$ was used to generate the eroded design and $\mu^d = 0.35$ to generate the dilated design. For the intermediate design $x^i$, $\mu^i = 0.5$ was used. This led to the following relation between the minimum length-scale $r_{min}$ and the filter radius $r_{fil}$ for the solid and void phase:

$$r_{min} = 2 * r_{fil} * 1.15$$

The local volume constraint was applied on the dilated design $x^d$, with the local volume fraction set to 0.35, the filter radius of 3.5 and p-mean penalty of 16.

A connectivity constraint, $c(x^d)$, based on the effective stiffness constraints, was advantageously added to the formulation. The mathematical design variable field was inverted with $x_{inv} = 1 - x$. Thereafter, an inverted filtered and dilated variable field was created, i.e., $\tilde{x}_{inv}$ and $x^d_{inv}$, respectively. A compression load and a minimum stiffness (or maximum compliance) constraint was applied in the form $U(x^d_{inv}) \le \gamma$. With $\gamma$, a user defined parameter which needed to be tuned. $U(x^d_{inv})$ is the strain energy of the inverted dilated density field in $\Omega_{implant}$. Decreasing $\gamma$ would increase the size of the void regions in the final design and increase the likelihood of interconnected void spaces to facilitate the removal of unsolidified powder during the additive manufacturing process. Finally, the Poisson's ratio for titanium was set to 0.3, the E-modulus to 110 GPa and the Solid Isotropic Material and Penalization method penalty and homogeneous initial condition were fixed at 3.0 and 0.35, respectively.

The optimization was run for 24 hours on a computational cluster using 1000 cores in parallel. The final design should at least exhibit a discreteness measure that was lower than or close to 3%. This value is considered to be an acceptable convergence to a solid/void design.

The resulting topologically optimized designs (TOD's) from the optimization process, called 'as-designed', have a voxelized surface, because of the voxel-based discretization. Subsequently, the 'as-built' design was achieved by applying an 'Iso volume' filter to the intermediate design and exported to a manufacturing file. The 'as-built' design was used for additive manufacturing and subsidence risk assessment. Morphological properties, i.e. porosity, pore interconnectivity (isolated pores) and pore diameter distributions of the 3D implant designs, were quantified.

A subsidence risk assessment was carried out on the 'as built' geometry using a commercial explicit FE solver. Non-linear geometric effects and a general contact formulation were included. The general contact was modelled between the vertebrae and the implant with a tangential friction coefficient of 0.8 . The bone tissue was modelled as a ductile material, using a rate-independent elasto-plastic material model that is capturing the asymmetric tension-compression yield and post-yield behaviour of bone (Figure 6b). Each FE model was meshed with ten-node tetrahedral elements, with an average element edge length of 2.0 mm for the vertebrae (selected after a mesh sensitivity study) and 0.5 mm for the cages. For both patients, three FE models were built to model the OTS, ACD and TOD cages, respectively. The models include the patient's FSU with the patient-specific calibration, segmentation, and material mapping. A Follower Load (FL) of 310 N and 1250 N for patient 1 and 2 were selected. The FLs were applied uniformly distributed on the superior endplate of the superior vertebra, such that the direction of the load is in line with the physiological alignment of the FSU (Figure 6a). The inferior endplate of the inferior vertebra was constrained in all translations (Figure 6a). The models were solved on a computational cluster using 64 cores in parallel.

For each FE model, subsidence risk was quantified as the degree of overloading of the vertebrae using a maximum principal strain limit of 1.5% and a minimum principal strain limit of -2.0%. For each element '$i$' in the bone domain, the factor of fracture risk ($FFR_i$) was calculated using:

$$FFR_i = max\left(\frac{\varepsilon_{max,i}}{1.50\%}, \frac{\varepsilon_{min,i}}{-2.00\%}\right)$$

where $\varepsilon_{max,i}$ is the maximum principal strain of element $i$, in the centroid of the element and $\varepsilon_{min,i}$ the minimum principal strain of element $i$, in the centroid of the element.

A prototype TOD design for patient 1 was additively manufactured using Titanium powder with a particle size range from 10 to 45 $\mu$m. Fixtures were created to fit the TOD top and bottom surfaces. Mechanical testing was performed using a static axial compression test.

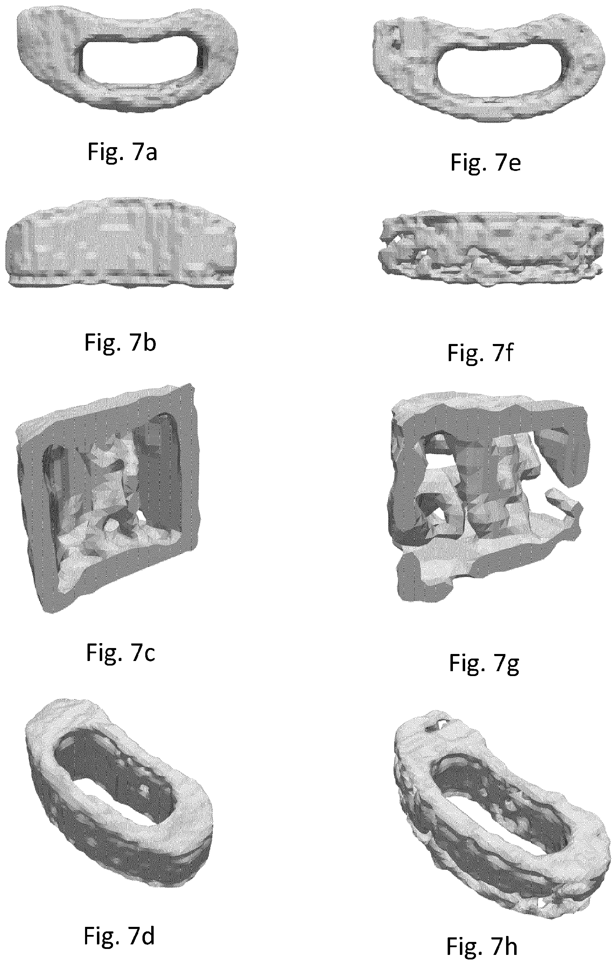

The resulting TODs for both patients are illustrated in Figure 7 (Patient 1: Figure 7a-7d, Patient 2; Figure 7e-7h). A 'box-like' design emerges with porous walls and hollow in the inside with several struts found internally. The outside shape of both TODs differs due to the varying endplate shapes of the vertebrae. The top and bottom of the implants, parts that are in contact with endplates, are almost without pores. Furthermore, the internal structure and the porosity in the walls is significantly different for both TODs, meaning that both TODs have similar macroscopic properties i.e., maximum contact area and box-like design to satisfy the same goal (reducing subsidence risk) but achieve this with different patient-specific micro-structures. The discreteness measure reached close to the desired 3% value with 3.4% and 3.3% for patient 1 and 2, respectively. The minimum strut size set was achieved, and no isolated pores were found, which made the TODs manufacturable.

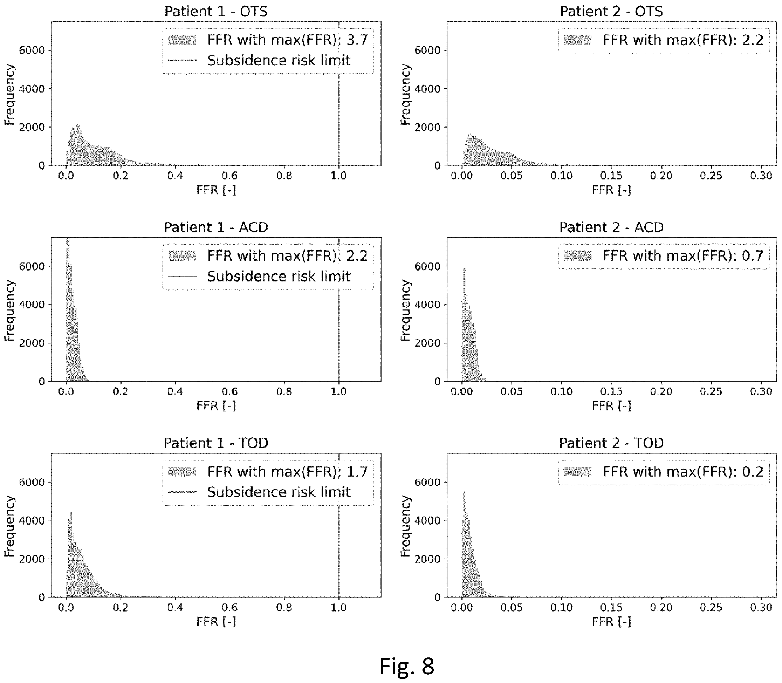

Histograms of the obtained FFR values for the different designs are illustrated in Figure 8. A decrease in subsidence risk from the OTS to the ACD and the TOD for both patients is observed. Furthermore, the reduction in subsidence risk is higher for patient 2, patient with normal bone quality. Specifically, for patient 1, subsidence risk is reduced by 54% compared to the OTS implant and by 91 % for patient 2.

**Claims**

1. A computer-implemented method (100) for designing an optimized patient-specific implant model (1) in view of its manufacturing comprising the following steps:

    a. define in a patient-specific representation of the region to receive the implant (3), at least a first bone domain (3a), a second bone domain (3b) and between the first and second bone domains an implant domain (3c), wherein the first bone domain (3a), the second bone domain (3b) and the implant domain (3c) form the optimization domain;
    b. create an initial implant model (1) that at least partially fills the implant domain (3c), wherein the boundaries of the initial implant model (1) are designed to fit the boundaries of the first and second bone domains (3a,3b) facing the implant domain (3c);
    c. optimize the implant model (1) by applying optimization, advantageously topology optimization or artificial intelligence-based optimization, until at least one of several termination criteria is reached, wherein the optimization is based on the mechanical response of at least of one of the first bone domain or the second bone domain (3a,3b) to mechanical load, in particular the subsidence risk.

2. Method according to claim 1, wherein in step c the implant model (1) is iteratively optimized by applying topology optimization until at least one of several termination criteria is reached, wherein in each iteration the mechanical response of at least of one of the first bone domain or the second bone domain (3a,3b) to mechanical load, in particular the subsidence risk, is determined and used for optimizing the implant model.

3. Method according to claim 1 or 2, wherein the mechanical load comprises shear loads, rotational loads, tension and/or compression load.

4. Method according to any one of the preceding claims, wherein the mechanical load comprises daily living loads and loads of higher intensity than daily living loads, wherein the intensity of the daily living loads are determined based on the weight of the patient.

5. Method according to any one of the claims 2 to 4, wherein the value of the objective function comprises the compliance, stiffness, or load bearing capacity of the optimization domain.

6. Method according to any one of preceding claims, wherein the strain response, in particular the minimum and maximum principal strains, or the stress response, in particular principal stress, of von Mises stress, or the energy response, in particular strain energy, of the first bone domain or/and of the second bone domain (3a,3b) are constrained.

7. Method according to any one of the preceding claims, wherein the minimum and maximum volume or local volume of the implant domain (3c) is constrained.

8. Method according to any one of the claims 2 to 7, wherein the topology optimization formulation comprises a manufacturability constraint for the implant domain (3c).

9. Method according to any one of the preceding claims, wherein the minimum diameter of the implant domain structures, for example struts, is constrained.

10. Method according to any one of the preceding claims, wherein the patient-specific image (3) is used to identify at least one tissue characteristic at one or more locations inside the first bone domain (3a) and/or the second bone domain (3b), wherein the at least one tissue characteristic comprises tissue density, tissue elasticity, tissue compliance, tissue stiffness and/or tissue strength, and wherein the at least one tissue characteristic is used to determine the mechanical response of the first bone domain (3a) and/or the second bone domain (3b) to mechanical load, and wherein the implant model (3c) is advantageously configured to contact a portion of the first bone domain and of the second bone domain (3a,3b) having a relatively higher tissue density compared to non-contacted portions of the first and second bone domains.

11. Method according to any one of the preceding claims wherein the patient-specific image (3) is a Computed Tomography (CT) image, for example, an X-ray image, in particular an X-ray CT image, a positron emission tomography (PET) image, a magnetic resonance imaging (MRI) image, a bone density scan image, such as a DEXA image, or any patient-specific image that provides bone information.

12. Method according to any one of the preceding claims further comprising a step for generating manufacturing information for manufacturing the patient-specific implant.

13. Method according to any of the preceding claims, wherein the patient-specific implant (1) is a spinal implant, in particular a spinal fusion implant, and the first bone domain and the second bone domain (3a, 3b) represent two adjacent vertebrae and wherein the implant is advantageously a spinal fusion implant cage.

14. Method according to claim 13 further comprising a step of assessing the risk of subsidence of the optimized implant model (1) in at least one of the bone domains (3a,3b).

15. Method for training an artificial intelligence model for the design of optimized implant models, said method comprising the step of utilizing at least one optimized implant model, said optimized implant model being obtained in accordance with the method as defined in any one of claims 1 to 14, wherein said artificial model is a neural network, preferably a convolutional neural network, and wherein said optimized implant models are specifically configured for spinal implants, more preferably spinal fusion implants

Fig. 1

Fig. 2

Fig. 3a

Fig. 3b

Fig. 4a

Fig. 4b

Fig. 4c

Fig. 4d

Fig. 5a

Fig. 5b

Fig. 6a

Fig. 6b

Fig. 7a

Fig. 7b

Fig. 7c

Fig. 7d

Fig. 7e

Fig. 7f

Fig. 7g

Fig. 7h

Fig. 8

Europäisches
Patentamt
European
Patent Office
Office européen
des brevets

# EUROPEAN SEARCH REPORT

**Application Number**

EP 23 21 1455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 212 131 A1 (PERIER METZ CAMILLE [FR]; DUDA GEORG [DE]; CHECA SARA [DE]) 19 July 2023 (2023-07-19) * paragraphs [0009] - [0088] * * claims 1-17 * * figure 1 * | 1-14 | INV. A61F2/30 A61B34/10 A61F2/44 |
| X | CN 111 281 613 B (UNION HOSPITAL TONGJI MEDICAL COLLEGE HUAZHONG UNIV SCI & TECH) 15 February 2022 (2022-02-15) * paragraphs [0005] - [0038] * * claims 1-6 * | 1-14 | |
| X | US 2023/130184 A1 (TURNER ALEX [US] ET AL) 27 April 2023 (2023-04-27) * paragraphs [0036] - [0058] * * claims 21-40 * | 1-14 | |
| X | CN 115 252 234 A (UNIV NANJING) 1 November 2022 (2022-11-01) * paragraphs [0008] - [0037] * * claims 1-7 * | 1-9, 11-14 | |

TECHNICAL FIELDS
SEARCHED      (IPC)

A61F
A61B

The present search report has been drawn up for all claims

| Place of search | Date of completion of the search | Examiner |
|---|---|---|
| Munich | 17 April 2024 | Felkel, Bernd |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**Application Number**

EP 23 21 1455

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 23 21 1455

The Search Division considers that the present European patent application does not comply with the
requirements of unity of invention and relates to several inventions or groups of inventions, namely:

    1. claims: 1-14

       A computer-implemented method for designing an optimized
       patient-specific implant model in view of its manufacturing.
                        - - -

    2. claim: 15

       Method for training an artificial intelligence model for the
       design of optimized implant models.
                        - - -

## ANNEX TO THE EUROPEAN SEARCH REPORT
## ON EUROPEAN PATENT APPLICATION NO.

EP 23 21 1455

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2024

| Patent document cited in search report | | Publication date | Patent family member(s) | | Publication date |
|---|---|---|---|---|---|
| EP 4212131 | A1 | 19-07-2023 | EP | 4212131 A1 | 19-07-2023 |
| | | | WO | 2023135267 A1 | 20-07-2023 |
| CN 111281613 | B | 15-02-2022 | NONE | | |
| US 2023130184 | A1 | 27-04-2023 | AU | 2017225796 A1 | 13-09-2018 |
| | | | AU | 2021203401 A1 | 01-07-2021 |
| | | | BR | 112018067591 A2 | 15-01-2019 |
| | | | EP | 3422940 A1 | 09-01-2019 |
| | | | EP | 3868294 A1 | 25-08-2021 |
| | | | ES | 2877761 T3 | 17-11-2021 |
| | | | IL | 261328 A | 31-10-2018 |
| | | | JP | 2019514450 A | 06-06-2019 |
| | | | US | 2017252107 A1 | 07-09-2017 |
| | | | US | 2020038111 A1 | 06-02-2020 |
| | | | US | 2021212766 A1 | 15-07-2021 |
| | | | US | 2023130184 A1 | 27-04-2023 |
| | | | US | 2024148438 A1 | 09-05-2024 |
| | | | WO | 2017151949 A1 | 08-09-2017 |
| CN 115252234 | A | 01-11-2022 | NONE | | |

EPO FORM P0459

For more details about this annex : see Official Journal of the European Patent Office, No. 12/82